# EUROPEAN PATENT SPECIFICATION

(11) **EP 3 893 851 B1**
(45) Date of publication and mention of the grant of the patent: **18.09.2024**
(21) Application number: 19835733.7
(22) Date of filing: 12.12.2019
(51) Int. Cl.: A61K 9/20, A61K 9/28, A61K 31/606

(54) **SOLID ORAL PHARMACEUTICAL COMPOSITIONS FOR ADMINISTRATION OF MESALAZINE OR DERIVATIVES THEREOF**
FESTE ORALE PHARMAZEUTISCHE ZUSAMMENSETZUNGEN ZUR VERABREICHUNG VON MESALAZIN ODER DERIVATEN DAVON
COMPOSITIONS PHARMACEUTIQUES ORALES SOLIDES POUR L'ADMINISTRATION DE MÉSALAZINE OU DE DÉRIVÉS DE CELLE-CI

(30) Priority: 14.12.2018 IT 201800011120
(43) Date of publication of application: 20.10.2021
(73) Proprietor: DPL Pharma S.p.A., 20089 Rozzano (MI) (IT)
(72) Inventor: PEDRANI, Massimo, 6815 Melide (CH); CONTI, Chiara, 29010 Vigolo Marchese (PC) (IT); GIAMMILLARI, Salvatore Agostino, 20089 Rozzano (MI) (IT); MACCARI, Giuseppe, 27058 Voghera (PV) (IT)
(74) Representative: Bianchetti & Minoja with Trevisan & Cuonzo IPS SRL
(86) International application number: PCT/IB2019/060689
(87) International publication number: WO 2020/121232

(56) References cited:
- EP-A1- 2 468 264
- WO-A1-2017/125856
- WO-A2-2009/047802

## Description

The present invention relates to solid oral pharmaceutical compositions for the site- and time-specific administration of mesalazine, the salts thereof or an analogue thereof selected from sulphasalazine, olsalazine, balsalazide and salazopyrin. The formulations according to the invention comprise mesalazine, the salts thereof or an analogue thereof selected from sulphasalazine, olsalazine, balsalazide and salazopyrin in a core consisting of a monolithic matrix comprising at least one low/average viscosity hydroxypropyl methylcellulose, at least one medium/high viscosity hydroxypropyl methylcellulose, one or more methacrylic acid copolymers soluble at pH ≥ 5.5 and an outer coating of said core consisting of a layer comprising ethylcellulose or of a gastroresistant layer or of a layer comprising ethylcellulose which in turn is coated with gastroresistant polymers.

### Prior art

Mesalazine or 5-aminosalicylic acid (5-ASA) is a medicament used in the acute and chronic treatment of inflammatory bowel disease (IBD), in particular in maintaining and/or inducing remission in patients suffering from mild/moderate active ulcerating colitis. Mesalazine derivatives or analogues comprise sulphasalazine, olsalazine, balsalazide and salazopyrin.

Mesalazine is available on the market in oral (coated tablets, slow-release tablets) or rectal pharmaceutical forms (suppositories, ready-to-use enemas, foam enemas, single-dose enemas, slow-release enemas). Slow-release oral and rectal technologies were devised to prolong the effect of the medicament and allow fewer daily administrations of tablets or enemas.

An ideal pharmaceutical form should release mesalazine into contact with the inflamed intestinal mucosa and limit its systemic bioavailability. In practice, mesalazine is absorbed by the intestinal mucosa, especially in the first part of the small intestine.

Mainly for this reason, the tablets are coated, so as to release the mesalazine into the last part of the small intestine and the colon. This result can be obtained by various technologies, for example by using pH-sensitive polymer matrices able to release mesalazine only in proximity to the colon.

In particular, some methacrylic acid copolymers (Eudragit), used as a coating in modern oral preparations, release the medicament when the pH of the external environment is between 5.5 and 7.2-7.5, the pH values being measured in the intestinal lumen, in the part of the colon affected by ulcerating colitis.

Gastroenteric-release products release all of the product within an hour of solubilisation of the film-coating. Examples of said systems are described in WO 2017/184566, WO 2017/072050 and WO 01/66094.

Multi-particulate systems, which are described, for example, in EP 0 629 398, EP 0 453 001, WO2017156214, WO200885484, US 2010/0136125, US20060210631 and WO 2006/102446, are characterised by pH-dependent release or reservoir systems without a matrix.

Matrix systems for the release of medicaments with two or three ingredients were described in WO 0076481, wherein mesalazine is incorporated in a lipophilic matrix dispersed in a hydrophilic matrix, or in three matrices (lipophilic, hydrophilic and amphiphilic), to delay and prolong the dissolution. A matrix system for the release of medicaments with two ingredients, wherein mesalazine is incorporated in a hydrophilic and amphiphilic matrix, has also been described (US2004213844). A gastroresistant polymer film gives rise to pH-dependent dissolution of the tablet at pH 7 in the terminal ileum. The monolithic matrix presents the drawback of possible irregular releases in the colonic tract, and the tablet is sometimes expelled before the medicament has been released. Other monolithic matrix systems are described in EP 2 468 264, US 2017/0143743, WO 2017/125856 and EP 1 321 368.

The known formulations do not provide the ideal solution to the problem of gradual, constant colonic release of the medicament continuing for several hours so as to guarantee homogeneous distribution, a reproducible release profile, and a very low coefficient of relative standard deviation.

### Description of the invention

It has now been found that the drawbacks of the known formulations of mesalazine can be eliminated by using complex matrices, consisting of a combination of several polymers having different characteristics.

In particular, it has been found that by combining at least two types of hydroxypropyl methylcellulose having different viscosities with methacrylic acid copolymers soluble at pH ≥ 5.5, formulations as claimed that overcome the limitations of the previously known formulations can be prepared. The formulations described in EP 2 468 264 possess a release profile characterised by a burst effect that is not found in the formulations according to the invention, which exhibit less variability of the dissolution profiles over time, with very low relative standard deviation (RSD) values, always under 3.

The solid oral controlled-release pharmaceutical compositions according to the invention comprise a core containing mesalazine, the salts thereof or an analogue thereof selected from sulphasalazine, olsalazine, balsalazide and salazopyrin and an outer coating of said core, wherein:
a) the core consists of:
   a monolithic matrix containing mesalazine, a salt thereof or an analogue thereof, at least one hydroxypropyl methylcellulose having a viscosity ranging between 3 and 5000 mPa.s 2 % in H₂O at 20°C, at least one hydroxypropyl methylcellulose having a viscosity ranging between 13500 and 280000 mPa.s 2% in H₂O at 20°C, at least one or more methacrylic acid copolymers soluble at pH ≥ 5.5,
b) the coating consists of a layer comprising ethylcellulose or of a gastroresistant layer or of a layer comprising ethylcellulose which in turn is coated with gastroresistant polymers.

Mesalazine is the preferred active ingredient in all the different embodiments of the invention.

The coating consists of a layer comprising ethylcellulose or, in another embodiment of the invention, coating b) consists of a layer comprising ethylcellulose coated with gastroresistant polymers.

In yet another embodiment of the invention, the coating consists of a gastroresistant layer.

According to one embodiment of the invention, the methacrylic acid copolymer is associated with shellac.

The gastroresistant coating can be the conventional type, and typically comprises methacrylic acid copolymers soluble at pH ≥ 5.5. Examples of said copolymers are available on the market (Eudragit). Preferably the combination of polymethacrylate L100 with polymethacrylate S100 at the ratio of 1:10 - 10:1 (preferably 1:1); or L 100/55 soluble at pH ≥ 5.5; or shellac; or cellulose acetate phthalates/succinates are used.

In the compositions according to preferred embodiments of the invention, the hydroxypropyl methylcellulose having a viscosity ranging between 3 and 5000 mPa.s 2% in H₂O at 20°C constitutes 1 to 20% of the weight of the core, the hydroxypropyl methylcellulose having a viscosity ranging between 13500 and 280000 mPa.s 2% in H₂O at 20°C constitutes 1 to 20% of weight of the matrix, and the methacrylic copolymer constitutes 0.1 to 20% of the weight of the core.

Hydroxypropyl methylcellulose having a viscosity ranging between 3 and 5000 mPa.s 2% in H₂O at 20°C is available on the market under the names of Methocel K3LV, E 5 Premium, K100 LV and K4M.

Hydroxypropyl methylcellulose having a viscosity ranging between 16500 and 280000 mPa.s 2% in H₂O at 20°C is available on the market under the names of Methocel K15M, K100 M and K200M.

Ethylcellulose is present in the core-coating layer in percentages ranging from 1% to 20% of the weight of the core, preferably 5%.

The amounts of mesalazine in the compositions according to the invention can range from 250 to 1600 mg per dosage unit.

The matrix core can comprise conventional excipients such as diluents (microcrystalline cellulose, starches, sugars, hydrated and anhydrous mono/dibasic phosphate/sodium phosphate salts), binders (PVP, starches, cellulose, dextrins, maltodextrins, low-viscosity cellulose), glidants (colloidal silicon dioxides), flow agents (talc), lubricants (Mg stearate, fumaryl stearate, stearic acid, glyceryl behenate), disintegrating agents (croscarmellose, sodium starch glycolate, crosslinked polyvinylpyrrolidone, starches) and other functional excipients (waxes, polycarbophil, carbomer, glycerides).

The matrix is prepared by processes of partition and direct compression, dry granulation, compacting, wet granulation, melting and extrusion.

Powders, granules, microgranules, pellets, mini-tablets, tablets, capsules, sachets and sticks can thus be obtained.

The resulting matrix/mini-matrix can then be coated with a gastroresistant film containing pH-dependent polymers that prevent release for at least 2 hours under pH conditions < 1.2-5.5. The following can be used for this purpose: pH-dependent methacrylic acid copolymers soluble at pH ≥ 5.5 (L 100-55/L 30 D-55); pH-dependent methacrylic acid copolymers soluble at pH 6.0-7.0 (L 100/L 12.5); pH-dependent methacrylic acid copolymers soluble at pH ≥ 7.0 (S 100/S 12.5/FS 30D); shellac; cellulose acetate phthalate; cellulose succinate.

At a third stage, a core coating can be applied which is alternative and/or additional to and beneath the gastroresistant coating with pH-independent polymers (ethylcellulose or hydroxypropyl methylcellulose with different viscosities), which act as membranes delaying the passage of the ingredient loaded into the matrix/mini-matrix core following contact with biological fluids.

The matrix is coated with a quantity of polymer sufficient to guarantee that it remains intact in gastric and enteric juices for at least 2-4 hours before the release of the active ingredient from the core (lag time). To reduce the impact of variable gastric voiding times, a further (pH-dependent) gastroresistant coating can be applied outside the (pH-independent) matrix core and outside the (pH-independent) cellulose film coating, to further delay contact between the biological fluids and the modified-release core (extended release).

In this way the system prevents early release during the stomach-jejunum transit time, initiating the modulated-release programme lasting up to 24 hours and ensuring homogeneous distribution of the active ingredient in the distal ileum and in the ascending, transverse and descending tracts of the large intestine.

The use of hydrophilic polymers with different rheological characteristics (viscosity/swelling properties) combined with pH-dependent and/or pH-independent polymers allows the release to be modulated for between 8 and 24 hours.

The system according to the invention offers the following advantages over the known monolithic and multiparticulate reservoir matrices:
- uniform release in the intestinal tract, specifically in the ascending, transverse and descending colon, maximising the site-specific effect and minimising plasma fluctuations, with fewer toxic effects and improved safety;
- homogeneous release profiles;
- gradual, constant, zero-order and first-order release;
- low burst effect;
- lower variability of dissolution profiles over time (low relative standard deviation);
- effective control of passage of biological fluids in the therapeutic system, by regulating the release of the medicament through the mini-matrix (pore forming).

The invention is described in greater detail in the examples below.

### EXAMPLE 1

1.2 Kg of mesalazine is loaded into a granulator with 105 g of hydroxypropyl methylcellulose (HPMC K4M), 54 g of hydroxypropyl methylcellulose (HPMC K100M), 2 g of polymethacrylate L100 and 2 g of polymethacrylate S100. The ingredients are mixed until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1472 mg. The resulting tablets are film-coated with a gastroresistant solution/suspension based on 36 g of polymethacrylate L100, 36 g of polymethacrylate S100, 8 g of talc, 3 g of titanium dioxide and 1 g of triethyl citrate, to obtain a tablet with a mean weight of 1556 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 30% after 1 hour, and not more than 35% after 2 hours; the value must be > 80% after 6 hours; and 100% after 10 hours.

### EXAMPLE 2

1.2 Kg of mesalazine is loaded into a granulator, and wet-granulated with an aqueous solution containing 83 g of PVP. The resulting granulate is dried, and then placed in a mixer with 106 g of hydroxypropyl methylcellulose (HPMC K4M), 53 g of hydroxypropyl methylcellulose (HPMC K100M) and 4 g of polymethacrylate L100-55. The ingredients are mixed until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1472 mg. The resulting tablets are film-coated with a solution/suspension of 27 g of ethylcellulose, 4 g of talc and 0.5 g of triethyl citrate. This is followed by a gastroresistant coating containing 45 g of polymethacrylate L100-55, 4 g of talc, 3 g of titanium dioxide and 0.5 g of triethyl citrate, to obtain a tablet with a mean weight of 1556 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 1% after 1 hour, and not more than 10% after 2 hours; not more than 30% after 6 hours; less than 50% after 8 hours; less than 80% after 10 hours; and 100% after 18 hours.

### EXAMPLE 3

1.2 Kg of mesalazine is loaded into a granulator with 15 g of hydroxypropyl methylcellulose (HPMC K 100lv), 35 g of hydroxypropyl methylcellulose (HPMC K100M), 2 g of polymethacrylate L100, 2 g of polymethacrylate S100 and 1 g of shellac. The ingredients are mixed until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1281 mg. The resulting tablets are then film-coated with a solution/suspension of 27 g of ethylcellulose, 8 g of talc, 3 g of titanium dioxide and 1 g of triethyl citrate, to obtain a tablet with a mean weight of 1320 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 5% after 1 hour, and not more than 10% after 2 hours; not more than 50% after 6 hours; less than 70% after 8 hours; less than 90% after 10 hours; and 100% after 18 hours.

### EXAMPLE 4

1.2 Kg of mesalazine is loaded into a granulator and wet-granulated with an aqueous solution containing 83 g of PVP. The resulting granulate is dried, and then placed in a mixer with 35 g of hydroxypropyl methylcellulose (HPMC K4M), 15 g of hydroxypropyl methylcellulose (HPMC K100M) and 4 g of polymethacrylate L100-55. The ingredients are mixed until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1363 mg. The resulting tablets are then film-coated with a solution/suspension of 27 g of ethylcellulose, 8 g of talc, 3 g of titanium dioxide and 1 g of triethyl citrate, to obtain a tablet with a mean weight of 1402 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 3% after 1 hour, and not more than 5% after 2 hours; not more than 45% after 6 hours; less than 70% after 8 hours; less than 90% after 10 hours; and 100% after 18 hours.

### EXAMPLE 5 (not part of the invention)

1.2 Kg of mesalazine is loaded into a granulator with 50 g of lactose monohydrate, 50 g of mannitol, 15 g of hydroxypropyl methylcellulose (HPMC K 100lv), 35 g of hydroxypropyl methylcellulose (HPMC K100M), 2 g of polymethacrylate RL100, 2 g of polymethacrylate RS100 and 1 g of shellac. The ingredients are mixed until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1380 mg. The resulting tablets are then film-coated with a solution/suspension of 27 g of ethylcellulose, 8 g of talc, 3 g of titanium dioxide and 1 g of triethyl citrate, to obtain a tablet with a mean weight of 1420 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 5% after 1 hour, and not more than 15% after 2 hours; not more than 55% after 6 hours; less than 70% after 8 hours; less than 90% after 10 hours; and 100% after 18 hours.

### EXAMPLE 6 (not part of the invention)

960 g of mesalazine is loaded into a granulator with 25 g of lactose and 25 g of microcrystalline cellulose, and wet-granulated with an aqueous solution containing 40 g of PVP. 15 g of hydroxypropyl methylcellulose (HPMC K 100lv), 35 g of hydroxypropyl methylcellulose (HPMC K15M), 1 g of polymethacrylate RL 100 and 1 g of polymethacrylate RS 100 are added in sequence to the resulting granulate after drying. The ingredients are mixed until a homogeneous dispersion of active ingredient in the matrices is obtained. 4 g of talc, 4 g of colloidal silicon dioxide and 5 g of magnesium stearate are then added in sequence. The mixture is then homogenised for at least 15 minutes. This mixture will form part of the first, controlled-release layer of the tablet.

240 g of mesalazine is loaded into a second granulator. 25 g of microcrystalline cellulose, 25 g of lactose monohydrate, 20 g of crospovidone, 20 g of croscarmellose, 1 g of magnesium stearate and 1 g of talc are added and homogeneously mixed. The mixture is then homogenised for at least 20 minutes. This mixture will form part of the second, immediate-release layer of the tablet. The two separate mixtures are then compressed to obtain a double-layer tablet weighing 1407 mg. The resulting tablets are then film-coated with a solution/suspension of 27 g of ethylcellulose, 8 g of talc, 3 g of titanium dioxide and 2 g of triethyl citrate, to obtain a tablet with a mean weight of 1487 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 15% after 1 hour, and not more than 25% after 2 hours; not more than 55% after 6 hours; less than 75% after 8 hours; less than 90% after 10 hours; and 100% after 18 hours.

### EXAMPLE 7

1.2 Kg of mesalazine is loaded into a granulator with 50 g of hydroxypropyl methylcellulose (HPMC K4M), 25 g of hydroxypropyl methylcellulose (HPMC K100M), 2 g of polymethacrylate L 100, 2 g of polymethacrylate S 100 and 1 g of shellac. The ingredients are mixed until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1306 mg. The resulting tablets are then film-coated with a gastroresistant solution/suspension based on 80 g of shellac, 2 g of hydroxypropyl methylcellulose E 5 Premium, 8 g of talc, 3 g of titanium dioxide and 1 g of triethyl citrate to obtain a mini-tablet with a mean weight of 1390 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 30% after 1 hour, and not more than 35% after 2 hours; the value must be > 80% after 6 hours; and 100% after 10 hours.

### EXAMPLE 8

750 g of mesalazine is loaded into a granulator and wet-granulated with an aqueous solution containing 50 g of PVP. After drying, 65 g of hydroxypropyl methylcellulose (HPMC K4M), 33 g of hydroxypropyl methylcellulose (HPMC K100M), 2.5 g of polymethacrylate L 100 and 2.5 g of polymethacrylate S 100 are added to the resulting granulate. The ingredients are mixed until a homogeneous dispersion of active ingredient in the matrices is obtained. 5 g of talc, 5 g of colloidal silicon dioxide and 7 g of magnesium stearate are then added in sequence. The mixture is then homogenised for at least 15 minutes. The mixture is then compressed to obtain a mini-tablet weighing 92 mg. The resulting mini-tablets are then film-coated with a gastroresistant solution of 33 g of polymethacrylate L 100, 33 g of polymethacrylate S 100, 35 g of talc, 12 g of titanium dioxide and 7 g of triethyl citrate, to obtain a mini-tablet with a mean weight of 104 mg. When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 60 minutes; when subjected to the dissolution test at pH ≥ 7.2 they exhibit the following release profile: not more than 30% after 60 minutes; not more than 35% after 120 minutes, not less than 80% after 360 minutes; the value must be 100% after 18 hours.

### EXAMPLE 9

750 g of mesalazine is loaded into a granulator and wet-granulated with an aqueous solution containing 50 g of PVP. After drying, 65 g of hydroxypropyl methylcellulose (HPMC K 4M), 33 g of hydroxypropyl methylcellulose (HPMC K 100M) and 5 g of polymethacrylate L 100-55 are added to the resulting granulate. The ingredients are mixed until a homogeneous dispersion of active ingredient in the matrices is obtained. 5 g of talc, 5 g of colloidal silicon dioxide and 7 g of magnesium stearate are then added in sequence. The mixture is then homogenised for at least 15 minutes. The mixture is then compressed to obtain a mini-tablet weighing 92 mg. The mini-tablets thus obtained are then film-coated with a solution/suspension of 60 g of ethylcellulose and 3.5 g of triethyl citrate; then further film-coated with a gastroresistant solution of 66 g of polymethacrylate L 100-55, 35 g of talc, 12 g of titanium dioxide and 3.5 g of triethyl citrate to obtain a mini-tablet with a mean weight of 110 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 60 minutes; when subjected to the dissolution test at pH ≥ 7.2 they exhibit the following release profile: not more than 5% after 60 minutes; not more than 10% after 120 minutes, and not less than 20% after 360 minutes; the value must be 100% after 18 hours.

### EXAMPLE 10

250 g of mesalazine is loaded into a granulator and wet-granulated with an aqueous solution containing 10 g of PVP. After drying, 5 g of hydroxypropyl methylcellulose (HPMC K 4M), 3 g of hydroxypropyl methylcellulose (HPMC K 100M), 2.5 g of polymethacrylate L 100 and 2.5 g of polymethacrylate S 100 are added to the resulting granulate. The ingredients are mixed until a homogeneous dispersion of active ingredient in the matrices is obtained. 5 g of talc, 5 g of colloidal silicon dioxide and 7 g of magnesium stearate are then added in sequence. The mixture is then homogenised for at least 15 minutes. The mixture is then compressed to obtain a mini-tablet weighing 28.1 mg. The resulting mini-tablets are film-coated with a solution/suspension of 25 g of ethylcellulose, 7 g of triethyl citrate, 1 g of talc and 1 g of titanium dioxide to obtain a mini-tablet with a mean weight of 31.5 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 1 hour; when subjected to the dissolution test at pH ≥ 7.2 they exhibit the following release profile: not more than 5% after 1 hour; not more than 10% after 2 hours, not more than 70% after 6 hours; the value must be ≥ 80% after 8 hours; and must reach 100% after 18 hours.

### EXAMPLE 11

250 g of mesalazine is loaded into a granulator and wet-granulated with an aqueous solution containing 10 g of PVP. After drying, 4 g of hydroxypropyl methylcellulose (HPMC K 4M), 2 g of hydroxypropyl methylcellulose (HPMC K 100M) and 5 g of polymethacrylate L 100-55 are added to the resulting granulate. The ingredients are mixed until a homogeneous dispersion of active ingredient in the matrices is obtained. 5 g of talc, 5 g of colloidal silicon dioxide and 7 g of magnesium stearate are then added in sequence. The mixture is then homogenised for at least 15 minutes. The mixture is then compressed to obtain a mini-tablet weighing 22.9 mg. The resulting mini-tablets are film-coated with a solution/suspension of 25 g of ethylcellulose, 7 g of triethyl citrate, 1 g of talc and 1 g of titanium dioxide to obtain a mini-tablet with a mean weight of 26.3 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 1 hour; when subjected to the dissolution test at pH ≥ 7.2 they exhibit the following release profile: not more than 5% after 1 hour; not more than 15% after 2 hours, not more than 75% after 6 hours; the value must be ≥ 80% after 8 hours; and must reach 100% after 18 hours.

### EXAMPLE 12

187.5 g of mesalazine is loaded into a granulator and wet-granulated with an aqueous solution containing 12.99 g of PVP. After drying, 16.7 g of hydroxypropyl methylcellulose (HPMC K 4M), 8.35 g of hydroxypropyl methylcellulose (HPMC K 100M), 0.53 g of polymethacrylate L 100 and 0.53 g of polymethacrylate S 100 are added to the resulting granulate. The ingredients are mixed until a homogeneous dispersion of active ingredient in the matrices is obtained. 1.25 g of talc, 1.26 g of colloidal silicon dioxide and 1.89 g of magnesium stearate are then added in sequence. The mixture is then homogenised for at least 15 minutes. The mixture is then compressed to obtain a mini-tablet weighing 23.1 mg. The resulting mini-tablets are film-coated with a gastroresistant solution/suspension of 23.76 g of polymethacrylate L 100, 23.76 g of polymethacrylate S 100, 2.3 g of triethyl citrate, 35.56 g of talc, 14.25 g of titanium dioxide and 2.84 g of iron oxide to obtain a mini-tablet with a mean weight of 35.5 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 1 hour; when subjected to the dissolution test at pH ≥ 7.2 they exhibit the following release profile: not more than 20% after 1 hour; not more than 30% after 2 hours, and not less than 80% after 6 hours; the value must be ≥ 90% after 8 hours; and must reach 100% after 18 hours.

### EXAMPLE 13

187.5 g of mesalazine is loaded into a granulator and wet-granulated with an aqueous solution containing 12.99 g of PVP. After drying, 16.7 g of hydroxypropyl methylcellulose (HPMC K 4M), 8.35 g of hydroxypropyl methylcellulose (HPMC K 100M), 0.53 g of polymethacrylate L 100 and 0.53 g of polymethacrylate S 100 are added to the resulting granulate. The ingredients are mixed until a homogeneous dispersion of active ingredient in the matrices is obtained. 1.25 g of talc, 1.26 g of colloidal silicon dioxide and 1.89 g of magnesium stearate are then added in sequence. The mixture is then homogenised for at least 15 minutes. The mixture is then compressed to obtain a mini-tablet weighing 23.1 mg. The resulting mini-tablets are film-coated with a solution/suspension of 16.28 g of ethylcellulose, 4.07 g of triethyl citrate, 2.21 g of titanium dioxide and 0.44 g of iron oxide to obtain a mini-tablet with a mean weight of 25.4 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 1 hour; when subjected to the dissolution test at pH ≥ 7.2 they exhibit the following release profile: not more than 10% after 1 hour; not more than 20% after 2 hours, not more than 70% after 6 hours; the value must be ≥ 80% after 8 hours; and must reach 100% after 18 hours.

### EXAMPLE 14 (not part of the invention)

750 g of mesalazine is loaded into a granulator and wet-granulated with an aqueous solution containing 50 g of PVP. After drying, 65 g of hydroxypropyl methylcellulose (HPMC K 4M), 33 g of hydroxypropyl methylcellulose (HPMC K 100M), 2 g of polymethacrylate RL 100, 2 g of polymethacrylate RS 100 and 1 g of shellac are added to the resulting granulate. The ingredients are mixed until a homogeneous dispersion of active ingredient in the matrices is obtained. 5 g of talc, 5 g of colloidal silicon dioxide and 7 g of magnesium stearate are then added in sequence. The mixture is then homogenised for at least 15 minutes. The mixture is then compressed to obtain a mini-tablet weighing 92 mg. The resulting mini-tablets are then film-coated with a gastroresistant solution/suspension of 60 g of shellac, 10 g of hydroxypropyl methylcellulose E 5 Premium, 35 g of talc, 12 g of titanium dioxide and 7 g of triethyl citrate to obtain a mini-tablet with a mean weight of 104 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 60 minutes; when subjected to the dissolution test at pH ≥ 7.2 they exhibit the following release profile: not more than 25% after 60 minutes; not more than 40% after 120 minutes, not less than 80% after 360 minutes; the value must be 100% after 18 hours.

### EXAMPLE 15

750 g of mesalazine is loaded into a granulator and wet-granulated with an aqueous solution containing 50 g of PVP. After drying, 33 g of hydroxypropyl methylcellulose (HPMC K 100 lv), 65 g of hydroxypropyl methylcellulose (HPMC K 100M), 2.5 g of polymethacrylate L 100 and 2.5 g of polymethacrylate S 100 are added to the resulting granulate. The ingredients are mixed until a homogeneous dispersion of active ingredient in the matrices is obtained. 5 g of talc, 5 g of colloidal silicon dioxide and 7 g of magnesium stearate are then added in sequence. The mixture is then homogenised for at least 15 minutes. The mixture is then compressed to obtain a mini-tablet weighing 92 mg. The resulting mini-tablets are then film-coated with a gastroresistant solution of 33 g of polymethacrylate L 100, 33 g of polymethacrylate S 100, 30 g of talc, 10 g of titanium dioxide and 4 g of triethyl citrate, to obtain a mini-tablet with a mean weight of 103 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 1% after 60 minutes; when subjected to the dissolution test at pH ≥ 7.2 they exhibit the following release profile: not more than 35% after 60 minutes; not more than 45% after 120 minutes, not less than 85% after 360 minutes; the value must be 100% after 18 hours.

Table 1 below summarises the qualitative and quantitative compositions of Examples 1-15.

**Table 1**

| **Active ingredient** | Mesalazine F1 | Mesalazine F2 | Mesalazine F3 | Mesalazine F4 | Mesalazine F5 | Mesalazine F6 (not part of the invention) | Mesalazine F7 |
|---|---|---|---|---|---|---|---|
| **Ingredients of MR core** | | | | | | | |
| | 1200 | 1200 | 1200 | 1200 | 1200 | 960 | 1200 |
| Lactose monohydrate | | | | | 50 | 25 | |
| Microcrystalline cellulose | | | | | 50 | 25 | |
| HPMC 100 lv | | | 15 | | 15 | 15 | |
| HPMC K4 M | 105 | 106 | | 35 | | | 50 |
| HPMC K15 M | | | | | 35 | 35 | |
| HPMC K100 M | 54 | 53 | 35 | 15 | | | 25 |
| Eudragit L100 / 55 | | 4 | | 4 | | | |
| Eudragit L 100 | 2 | | 2 | | | | 2 |
| Eudragit S 100 | 2 | | 2 | | | | 2 |
| Shellac | | | 1 | | | | 1 |
| Eudragit RL 100 | | | | | 2 | 1 | |
| Eudragit RS 100 | | | | | 2 | 1 | |
| Talc | 8 | 8 | 8 | 8 | 8 | 4 | 8 |
| Hydrated coll. silicon dioxide | 8 | 8 | 8 | 8 | 8 | 4 | 8 |
| PVP | | 83 | | 83 | | 40 | |
| Mg stearate | 10 | 10 | 10 | 10 | 10 | 5 | 10 |
| | 1472 | 1472 | 1281 | 1363 | 1380 | 1075 | 1306 |

| **Active ingredient** | Mesalazine F1 | Mesalazine F2 | Mesalazine F3 | Mesalazine F4 | Mesalazine F5 | Mesalazine F6 | Mesalazine F7 |
|---|---|---|---|---|---|---|---|
| **Ingredients of IR core** | | | | | | | |
| | | | | | | 240 | |
| Lactose monohydrate | | | | | | 25 | |
| Microcrystalline cellulose | | | | | | 25 | |
| Crosslinked PVP | | | | | | 20 | |
| Croscarmellose (AcDisol) | | | | | | 20 | |
| Talc | | | | | | 1 | |
| Mg stearate | | | | | | 1 | |
| | | | | | | | |
| Total | | | | | | 332 | |
| | | | | | | | |

| **Film-coating ingredients** | | | | | | | |
|---|---|---|---|---|---|---|---|
| | | | | | | | |
| Talc | 8 | 8 | 8 | 8 | 8 | 8 | 8 |
| Eudragit L 100 / 55 | | 45 | | | | | |
| Eudragit L 100 | 36 | | | | | | |
| Eudragit S 100 | 36 | | | | | | |
| Shellac | | | | | | | 80 |
| Ethylcellulose | | 27 | 27 | 27 | 27 | 27 | |

| | Mesalazine F1 | Mesalazine F2 | Mesalazine F3 | Mesalazine F4 | Mesalazine F5 | Mesalazine F6 | Mesalazine F7 |
|---|---|---|---|---|---|---|---|
| Titanium dioxide | 3 | 3 | 3 | 3 | 3 | 3 | 3 |
| Triethyl citrate | 1 | 1 | 1 | 1 | 2 | 2 | 1 |
| HPMC 5 premium | | | | | | | 2 |
| Total | 84 | 84 | 39 | 39 | 40 | 40 | 84 |
| Grand total | 1556 | 1556 | 1320 | 1402 | 1420 | 1487 | 1390 |

**Table 2 - Active ingredient of Mini-tablets**

| **Active ingredient** | Mesalazine* F8 | Mesalazine* F9 | Mesalazine* * F10 | Mesalazine*** F11 | Mesalazine**** F12 | Mesalazine**** F13 | Mesalazine* F14 | Mesalazine* F15 |
|---|---|---|---|---|---|---|---|---|
| **Ingredients of MR MiniCore** | | | | | | | | |
| | 75 | 75 | 25 | 20 | 18.75 | 18.75 | 75 | 75 |
| HPMC 100 lv | | | | | | | | 3.3 |
| HPMC K4 M | 6.5 | 6.5 | 0.5 | 0.4 | 1.670 | 1.670 | 6.5 | |
| HPMC K15 M | | | | | | | | 6.5 |
| HPMC K100 M | 3.3 | 3.3 | 0.3 | 0.2 | 0.835 | 0.835 | 3.3 | |
| Eudragit L100 / 55 | | 0.5 | | 0.5 | | | | |
| Eudragit L 100 | 0.25 | | 0.25 | | 0.053 | 0.053 | | 0.25 |
| Eudragit S 100 | 0.25 | | 0.25 | | 0.053 | 0.053 | | 0.25 |
| Shellac | | | | | | | 0.1 | |
| Eudragit RL 100 | | | | | | | 0.2 | |
| Eudragit RS 100 | | | | | | | 0.2 | |
| PVP | 5 | 5 | 0.1 | 0.1 | 1.299 | 1.299 | 5 | 5 |
| Talc | 0.5 | 0.5 | 0.5 | 0.5 | 0.125 | 0.125 | 0.5 | 0.5 |
| Mg stearate | 0.7 | 0.7 | 0.7 | 0.7 | 0.189 | 0.189 | 0.7 | 0.7 |
| Silicon dioxide | 0.5 | 0.5 | 0.5 | 0.5 | 0.126 | 0.126 | 0.5 | 0.5 |
| | 92 | 92 | 28.1 | 22.9 | 23.10 | 23.10 | 92 | 92 |
| | | | | | | | | |

| **Film-coating ingredients** | | | | | | | | |
|---|---|---|---|---|---|---|---|---|
| | | | | | | | | |
| Talc | 3.5 | 3.5 | 0.1 | 0.1 | 3.563 | | 3.5 | 3 |
| Eudragit L100 / 55 | | 6.6 | | | | | | |
| Eudragit L 100 | 3.3 | | | | 2.376 | | | 3.3 |
| Eudragit S 100 | 3.3 | | | | 2.376 | | | 3.3 |
| Shellac | | | | | | | 6 | |
| HPMC E 5 P | | | | | | | 1 | |
| Ethylcellulose | | 6 | 2.5 | 2.5 | | 1.628 | | |
| Titanium dioxide | 1.2 | 1.2 | 0.1 | 0.1 | 1.425 | 0.221 | 1.2 | 1 |
| Triethyl citrate | 0.7 | 0.7 | 0.7 | 0.7 | 2.376 | 0.407 | 0.7 | 0.4 |
| Iron dioxide | | | | | 0.284 | 0.044 | | |
| Total | 12 | 18 | 3.4 | 3.4 | 12.4 | 2.3 | 12 | 11 |
| Grand total | 104 | 110 | 31.5 | 26.3 | 35.5 | 25.4 | 105 | 103 |

| | | | | | | | | |
|---|---|---|---|---|---|---|---|---|
| Mesalazine* 16 mini-tablets 4 mm = 1200 mg Mesalazine** 48 mini-tablets 3 mm = 1200 mg Mesalazine*** 60 mini-tablets 3 mm = 1200 mg Mesalazine**** 64 mini-tablets 3 mm = 1200 mg | | | | | | | | |

## Claims

1. A controlled-release solid oral pharmaceutical composition comprising a core containing mesalazine, the salts thereof or an analogue thereof selected from sulphasalazine, olsalazine, balsalazide and salazopyrin, and an outer coating of said core, **characterised in that**:
a) the core consists of:
a monolithic matrix containing mesalazine, the salts or an analogue thereof, at least one hydroxypropyl methylcellulose having a viscosity ranging between 3 and 5000 mPa.s 2% in H₂O at 20°C, at least one hydroxypropyl methylcellulose having a viscosity ranging between 135000 and 280000 mPa.s 2% in H₂O at 20°C, at least one or more methacrylic acid copolymers soluble at pH ≥ 5.5,
b) the coating consists of a layer comprising ethylcellulose or of a gastroresistant layer.

2. A composition as claimed in claim 1 wherein the coating consists of a layer comprising ethylcellulose.

3. A composition as claimed in claim 1 or 2 wherein the coating consists of a layer comprising ethylcellulose coated with gastroresistant polymers.

4. A composition as claimed in claim 1 or 2 wherein the coating consists of a gastroresistant layer.

5. A composition as claimed in one or more of claims 1 to 4 wherein the gastroresistant coating comprises pH-dependent methacrylic acid copolymers soluble at pH ≥ 5.5; pH-dependent methacrylic acid copolymers soluble at pH 6.0-7.0; H-dependent methacrylic acid copolymers soluble at pH ≥ 7.0; shellac; cellulose acetate phthalate; cellulose succinate.

6. A composition as claimed in one or more of claims 1 to 5 wherein the hydroxypropyl methylcellulose having a viscosity ranging between 3 and 5000 mPa.s 2% in H₂O at 20°C constitutes 1 to 20% of the weight of the core, the hydroxypropyl methylcellulose having a viscosity ranging between 135000 and 280000 mPa.s 2% in H₂O at 20°C constitutes 1 to 20% of the weight of the matrix, and the methacrylic copolymer constitutes 0.1 to 2% of the weight of the core.

7. A composition as claimed in one or more of claims 1-6 wherein ethylcellulose is present in percentages ranging from 1 to 20% of the weight of the core.

8. A composition as claimed in claim 7 wherein ethylcellulose is present in a percentage of 5 % of the weight of the core.

## Patentansprüche

1. Feste orale pharmazeutische Zusammensetzung mit kontrollierter Freisetzung, umfassend einen Kern, der Mesalazin, die Salze davon oder ein Analogon davon, ausgewählt aus Sulfasalazin, Olsalazin, Balsalazid und Salazopyrin, enthält, und eine äußere Beschichtung des Kerns, **dadurch gekennzeichnet, dass**
a) der Kern aus dem Folgenden besteht:
einer monolithischen Matrix, die Mesalazin, die Salze oder ein Analogon davon, mindestens eine Hydroxypropylmethylcellulose mit einer Viskosität zwischen 3 und 5000 mPa.s 2% in H₂O bei 20°C, mindestens eine Hydroxypropylmethylcellulose mit einer Viskosität zwischen 135000 und 280000 mPa.s 2% in H₂O bei 20°C, mindestens ein oder mehrere Methacrylsäure-Copolymere, die bei einem pH-Wert ≥ 5,5 löslich sind, enthält,
b) die Beschichtung aus einer Schicht, die Ethylcellulose umfasst oder aus einer gastroresistenten Schicht besteht.

2. Zusammensetzung nach Anspruch 1, wobei die Beschichtung aus einer Schicht besteht, die Ethylcellulose umfasst.

3. Zusammensetzung nach Anspruch 1 oder 2, wobei die Beschichtung aus einer Schicht besteht, die Ethylcellulose, beschichtet mit gastroresistenten Polymeren, umfasst.

4. Zusammensetzung nach Anspruch 1 oder 2, wobei die Beschichtung aus einer gastroresistenten Schicht besteht.

5. Zusammensetzung nach einem oder mehreren der Ansprüche 1 bis 4, wobei die gastroresistente Beschichtung pH-abhängige Methacrylsäure-Copolymere, die bei einem pH-Wert von ≥ 5,5 löslich sind; pH-abhängige Methacrylsäure-Copolymere, die bei einem pH-Wert von 6,0-7,0 löslich sind; pH-abhängige Methacrylsäure-Copolymere, die bei einem pH-Wert von ≥ 7,0 löslich sind; Schellack; Celluloseacetatphthalat; Cellulosesuccinat umfasst.

6. Zusammensetzung nach einem oder mehreren der Ansprüche 1 bis 5, wobei die Hydroxypropylmethylcellulose mit einer Viskosität im Bereich von 3 bis 5000 mPa.s 2% in H₂O bei 20°C 1 bis 20% des Gewichts des Kerns ausmacht, die Hydroxypropylmethylcellulose mit einer Viskosität im Bereich von 135000 bis 280000 mPa.s 2% in H₂O bei 20°C 1 bis 20% des Gewichts der Matrix ausmacht und das Methacryl-Copolymer 0,1 bis 2% des Gewichts des Kerns ausmacht.

7. Zusammensetzung nach einem oder mehreren der Ansprüche 1 bis 6, wobei Ethylcellulose in einem Prozentsatz von 1 bis 20% des Gewichts des Kerns vorhanden ist.

8. Zusammensetzung nach Anspruch 7, wobei Ethylcellulose in einem Prozentsatz von 5% des Gewichts des Kerns vorhanden ist.

## Revendications

1. Composition pharmaceutique orale solide à libération contrôlée comprenant un noyau contenant de la mésalazine, les sels de celle-ci ou un analogue de celle-ci choisi parmi la sulfasalazine, l'olsalazine, la balsalazide et la salazopyrine, et un enrobage extérieur dudit noyau, **caractérisée en ce que** :
a) le noyau est constitué :
d'une matrice monolithique contenant de la mésalazine, les sels ou un analogue de celle-ci, au moins une hydroxypropylméthylcellulose ayant une viscosité comprise entre 3 et 5 000 mPa.s 2 % dans le H₂O à 20 °C, au moins une hydroxypropylméthylcellulose ayant une viscosité comprise entre 135 000 et 280 000 mPa.s 2 % dans le H₂O à 20 °C, au moins un ou plusieurs copolymères d'acide méthacrylique solubles à un pH ≥ 5,5,
b) l'enrobage est constitué d'une couche comprenant de l'éthylcellulose ou d'une couche gastro-résistante.

2. Composition selon la revendication 1, dans laquelle l'enrobage est constitué d'une couche comprenant de l'éthylcellulose.

3. Composition selon la revendication 1 ou 2, dans laquelle l'enrobage est constitué d'une couche comprenant de l'éthylcellulose enrobée de polymères gastro-résistants.

4. Composition selon la revendication 1 ou 2, dans laquelle l'enrobage est constitué d'une couche gastro-résistante.

5. Composition selon une ou plusieurs des revendications 1 à 4, dans laquelle l'enrobage gastro-résistant comprend des copolymères d'acide méthacrylique dépendants du pH solubles à pH ≥ 5,5 ; des copolymères d'acide méthacrylique dépendants du pH solubles à pH 6,0-7,0 ; des copolymères d'acide méthacrylique dépendants du pH solubles à pH ≥ 7,0 ; de la gomme laque ; de l'acétophtalate de cellulose ; du succinate de cellulose.

6. Composition selon une ou plusieurs des revendications 1 à 5, dans laquelle l'hydroxypropylméthylcellulose ayant une viscosité comprise entre 3 et 5 000 mPa.s 2 % dans le H₂O à 20 °C constitue 1 à 20 % de la masse du noyau, l'hydroxypropylméthylcellulose ayant une viscosité comprise entre 135 000 et 280 000 mPa.s 2 % dans le H₂O à 20 °C constitue 1 à 20 % de la masse de la matrice et le copolymère méthacrylique constitue 0,1 à 2 % de la masse du noyau.

7. Composition selon une ou plusieurs des revendications 1 à 6, dans laquelle l'éthylcellulose est présente dans des pourcentages compris entre 1 à 20 % de la masse du noyau.

8. Composition selon la revendication 7, dans laquelle l'éthylcellulose est présente dans un pourcentage de 5 % de la masse du noyau.
